# EUROPEAN PATENT SPECIFICATION

(11) **EP 1 786 436 B1**
(45) Date of publication and mention of the grant of the patent: **14.11.2012**
(21) Application number: 05743430.0
(22) Date of filing: 29.04.2005
(51) Int. Cl.: A61K 31/70, A61P 11/00, A61P 9/00, A61P 19/00, A61K 31/7004

(54) **METHOD FOR IMPROVING VENTILATORY EFFICIENCY**
VERFAHREN FÜR VERBESSERUNG DER ATMUNGSEFFIZIENZ
PROCEDE POUR AMELIORER L'EFFICACITE VENTILATOIRE

(30) Priority: 29.04.2004 US 566584 P; 09.09.2004 US 608320 P
(43) Date of publication of application: 23.05.2007
(73) Proprietor: RiboCor, Inc., Minneapolis, MN 55428 (US)
(72) Inventor: MAC CARTER, Dean, J., Englewood, CO 80111 (US); ST. CYR, John, A., Coon Rapids, MN 55448 (US)
(74) Representative: Lucas, Brian Ronald
(86) International application number: PCT/US2005/015076
(87) International publication number: WO 2005/107768

(56) References cited:
- EP-A- 1 247 525
- WO-A-00/36915
- WO-A-99/48361
- WO-A-99/65476
- US-A- 4 767 785
- US-A- 5 140 045
- US-A- 6 159 943
- CARTES O ET AL: "D-ribose supplementation improves peak exercise capacity and ventilatory efficiency in heart failure patients" JOURNAL OF THE AMERICAN COLLEGE OF CARDIOLOGY, ELSEVIER, NEW YORK, NY, US, vol. 45, no. 3 Suppl A, 1 February 2005 (2005-02-01), page 185A, XP008095515 ISSN: 0735-1097
- OMRAN H ET AL: "D-Ribose improves diastolic function and quality of life in congestive heart failure patients: A prospective feasibility study" EUROPEAN JOURNAL OF HEART FAILURE 200310 NL, vol. 5, no. 5, October 2003 (2003-10), pages 615-619, XP002493102 ISSN: 1388-9842
- DATABASE CAPLUS [Online] GUAZZI M. ET AL.: 'Insulin ameliorates excercise ventilatory efficiency and oxygen uptake in patients with heart failure-type 2 diabets comorbidity', XP003009986 Retrieved from STN Database accession no. (2003:803172) & JOURNAL OF AMERICAN COLLEGE OF CARDIOLOGY vol. 42, no. 6, 2003, pages 1044 - 1050
- DATABASE CAPLUS [Online] CABAN J.: 'The effect of Insulin on Respiration in Tetanic Patients', XP003009987 Retrieved from STN Database accession no. (1961:14605) & POLSKIE ARCH. MED. WEWNETRZNEJ vol. 29, 1959, pages 1333 - 1340
- REINDL I ET AL: "Impaired ventilatory efficiency in chronic heart failure: Possible role of pulmonary vasoconstriction", AMERICAN HEART JOURNAL 1998 US, vol. 136, no. 5, 1998, pages 778-785, ISSN: 0002-8703
- SUN X -G ET AL: "Ventilatory efficiency during exercise in healthy subjects", AMERICAN JOURNAL OF RESPIRATORY AND CRITICAL CARE MEDICINE 20021201 US LNKD- DOI:10.1164/RCCM.2202033, vol. 166, no. 11, 1 December 2002 (2002-12-01), pages 1443-1448, ISSN: 1073-449X
- OIKAWA K ET AL: "Mechanism of the reduced ventilatory efficiency in healthy older men", CIRC J, vol. 67, no. Supplement 1, 2003, page 215, Japan ISSN: 1346-9843

## Description

### BACKGROUND OF THE INVENTION

Many subjects have sub-optimal pulmonary function as determined from an analysis of ventilatory efficiency, leading to fatigue and poor quality of life. Ventilatory efficiency is defined as ventilation per unit of CO₂ production, reflecting the ratio between breathing and effective perfusion of O₂ and CO₂ throughout the body. Included in the group with reduced ventilatory efficiency are those suffering from pulmonary conditions such as emphysema, cystic fibrosis, pulmonary fibrosis, chronic obstructive pulmonary disease, asthma and bronchitis. Even subjects with "normal" lungs can have poor pulmonary function for a variety of reasons. Persons with anemia or low O₂/CO, carrying capacity breath rapidly but ineffectively. Renal disease and exposure to high or low atmospheric pressure may also interfere with pulmonary function. Persons having reduced lung volume from scoliosis, spondylitis, surgery or trauma also do not maintain an optimal ventilation-to-perfusion ratio. Persons suffering from lung cancer often have both anemia and reduced lung volume due to tumors blocking ortions of the bronchial tree. A very large cohort of subjects with reduced pulmonary function are those suffering from cardiovascular disease including patients with stable coronary artery disease, myocardial ventricular hypertrophy, cardiomegaly, or congenital heart anomalies.

In the past, pulmonary function was estimated by measuring percent oxygen saturation of the blood or instant oxygen uptake (VO₂). While useful, these measurements are a photo of a point in time; useful to describe the state of the patient's pulmonary function under the testing conditions, but not able to predict function under differing conditions. A person at rest with normal oxygen saturation or uptake may encounter dyspnea under, for example, exercise conditions, when function under differing conditions. A person at rest with normal oxygen saturation or uptake may encounter dyspnea under, for example, exercise conditions, when oxygen demand is higher or under lower oxygen tension, when oxygen availability is lower. Ventilation efficiency (VE), on the other hand, reflects the actual condition of the lungs.

There exists a spectrum of deficit in ventilatory efficiency. Patients can present with reduced VE even before the diagnosis of a medical condition. These patients may include those with primary lung dysfunction because of emphysema, whether due to smoking or to genetic causes, asthma, chronic bronchitis and chronic obstructive pulmonary disorder. Patients with autoimmune diseases such as rheumatoid arthritis often develop "rheumatoid lung." Patients with low lung volume due to premature birth, scoliosis, spondylitis or subdevelopment due to lifelong inactivity also are at risk for pulmonary complications. Often, persons who consider themselves to be in good health with a good nutritional status are actually somewhat suboptimal in both parameters, rendering them at risk for developing medical conditions or predisposing them to fatigue. Those who would benefit from exercising are disinclined to do so.

An example of a particularly unique use for ventilatory efficiency measurement is in monitoring congestive heart failure (CHF) it has been found that patients with a poor VE have a significantly poorer prognosis for survival, than those CHF patients with a lower VE. Many patients suffering from moderate to advanced CHF show impairment of ventilatory efficiency. Patients at earlier stages of heart failure, Class I may actually have an elevation in their ventilatory efficiency. The majority of patients diagnosed as Class IV will have a significant deficit. However, as failure worsens, most patients will begin to have an impairment.

An advanced approach to treat and prevent pulmonary dysfunction is to recommend supplementation of key nutrients that will aid healing and enhance the physical state of the individual. Such nutritional formulations may be termed "dietary supplements," "functional foods" or "medical foods." In order to formulate an effective dietary supplement or functional or medical food, an understanding of the scientific basis behind the key ingredients is essential. Once a well-grounded recommendation toward dietary modification is made it can have a powerful influence on delay of onset of a medical condition, slowing of progression of the illness, hastening the recovery and continued maintenance of improved health in the individual afflicted with the medical condition.
Thus the need remains to select a supplement to be used in a method of improving VE in subjects with reduced VE.
Cartes et al, Journal of the American College of Cardiology, Vol. 45, No. 3 Suppl. A, 1 February 2005, discloses that D-ribose in heart failure patients improves VE.
EP-A-1247525 teaches that D-ribose is effective to relieve from dyspnea a subject suffering from acute mountain sickness. WO 99/65476 teaches that D-ribose should be administered for at least 5 days, preferably throughout the stay at high altitudes.
Omran et al, European Journal of Heart Failure, Vol. 5, No. 5, October 2003, discloses the use of D-ribose for the treatment of congestive heart failure.
US-A-4767785 discloses the use of a composition containing xylitol for the treatment of patients requiring ventilator support.
WO 00/36915 discloses the use of several compounds including D-ribose for the treatment of chronic obstructive airway diseases.
WO 99/48361 teaches the use of a xylitol/xylulose solution for the treatment of asthma.

### SUMMARY OF THE INVENTION

The present invention provides D-ribose for use in improving ventilatory efficiency in subjects suffering from autoimmune disease resulting in poor pulmonary function. The autoimmune disease is preferably rheumatoid lung

The D-ribose may be administered to a patient at least once a day in unit dosages of from two to ten grams. Preferably a unit dosage of two to ten grams of D-ribose is to be administered two or three times a day. Most preferably a unit dosage of five grams of D-ribose is given three times per day. The unit dosage may be dissolved in a suitable amount of water or may be ingested as a powder.

The administration should be continued for at least one week, but preferably should be continued long term, throughout the life of the subject.

The D-ribose of this invention is preferably dissolved in about eight ounces of liquid and ingested as a solution. Flavorings and other additives may be added to make the solution more palatable. D-Ribose in a unit dosage of one to 20 grams may be administered two to four times per day. Other supplements and medications are suggested to be co-administered in order to provide incremental enhancement of the method. These other supplements include vitamins and vasodilators.

### DETAILED DESCRIPTION OF THE INVENTION

The invention comprises D-ribose for the improvement of ventilatory efficiency as defined in the appended claims. D-Ribose is administered for at least one week, but preferably for a long term, or for the life of the subject. Supplements and medications that enhance the D-ribose effect on VE are suggested. Those supplements selected will have effects on metabolic pathways or physiological functions different from those of D-ribose and thus will have incremental benefit over the basic benefit of D-ribose alone. Improvement of VE results inherently in improvement of a subject's physical capability, enhances the subject's quality of life and may prevent or delay the development of medical conditions exacerbated by inaction. Therefore, in the present invention, when the term "pulmonary function" is used, it is understood to

include improvement of physical capability and enhancement of quality of life. When the term "subject benefitting from improved ventilatory efficiency" is used, it is understood to include both those subjects with reduced ventilatory efficiency and those subjects at risk of developing it.

D-ribose is a natural 5-carbon sugar found in every cell of the body. D-Ribose is the key ingredient in the compositions described in this invention. Other energy enhancers might be included that may increase the effect of D-ribose. Supplements that act by other mechanisms can be energy enhancers that would optimize the nutritional composition. For example, increasing a vessel's diameter by a vasodilator such as adenosine or nitrate would increase blood flow to reach outlying muscle tissue beds and thus improve the transport of D-ribose and nutrients to that tissue and thereby positively enhance physiological functions.

Ascorbic acid, otherwise known as vitamin C, is a water-soluble vitamin that is an essential nutrient. It plays a role in the detoxification of potentially damaging free radicals and may be the most important antioxidant in the watery extra-cellular environment of the body. L-carnitine has been shown to increase exercise capacity in both athletes and patients with angina, presumably by increasing the availability of fatty acids for oxidative metabolism. Pyruvate and creatine are also commonly used supplements for athletic enhancement. Folic acid (or folate) is vital for cell division and homeostasis due to the essential role of folate coenzymes in nucleic acid synthesis, methionine regeneration (from the remethylation of homocysteine), and in the shuttling, oxidation, and reduction of one-carbon units required for normal metabolism and regulation. Folate deficiency is thought to be one of the most common avitaminoses. Any of these supplements would be expected to enhance the D-ribose effect.

Normally, a wholesome diet is considered to provide sufficient amounts of these nutritive elements. D-ribose is known to be found only at low concentrations in foods, making the ingesting of optimal levels of ribose from the diet unlikely. Although D-ribose is synthesized from glucose in the body, the pathway is slow. The other pentoses are even lower in concentration in food. Vitamins and vasodilators suggested for improving ventilatory efficiency are available commercially, over the counter or by prescription. Supplementation with off-the-shelf multivitamins is common. It is of increased benefit to add at least vitamins and vasodilators to the methods of this invention.

The following examples are provided for illustrative purposes only.

### Example 1. Ventilatory efficiency in CHF (Reference example).

Ventilatory efficiency has been critically shown to be the most powerful, independent predictor of CHF patient survival. VE is determined by the linear, submax relationship between Minute Ventilation (V) and carbon dioxide output (VCO₂), V being on the "y axis" and the linear slope being determined using the linear regression model, y = a + bx, "b" representing the slope. The steeper the slope, the worse the ventilation efficiency of the patient.

Ventilation efficiency represents the degree of sympatho-excitation in the heart disease patient that reflects increased dead space in the lungs and augmented mechanoreceptor "drive" from the skeletal muscles. CHF patients with a VE slope greater than 36.9 have a significantly poorer prognosis for survival, as determined by Kaplan Meier graphics, than those CHF patients with a VE slope lower than 36.9.

Ventilation efficiency correlates with the level of cardiac preload or filling pressures to the heart. Higher filling pressures adversely affect pulmonary venous flow and cause pulmonary ventilation- to-perfusion mismatching, thus increasing the VE slope. VE slope has also been shown to correlate inversely with heart rate variability (HRV), a known predictor of sudden cardiac death in CHF patients.

### A. Ventilatory efficiency during exercise testing.

As an exemplar cohort of patients with reduced ventilatory efficiency, patients suffering from CHF were recruited. Patients having CHF were selected according to the following criteria:
- Male and female 48-84 years of age.
- Ejection fraction 30-72%.
- NY Class 111-IV (severe condition).
- Test and control groups matched for pre-operative volume status, cardiac medication, measured risk assessment.

The test group was administered D-ribose 15 grams tid for eight weeks; the controls received 15 grams Dextrose tid. All patients in this group underwent repeat cardiopulmonary exercise using a four-minute sub-maximal step protocol. Patients were tested on a step apparatus. Others in the study were tested on a treadmill with varied grade or on drug-driven exercise simulation for those patients unable to use the other two devices. Symptom-limited peak exercise performance with at least 80-85% of age related maximal heart rate was attempted with each patient. Upper extremity blood pressures was be obtained at every 2 minutes and also at peak exercise. V_{CO2} and V_{O2max} before and after exercise was be measured and VE calculated. The methodology is described in Circulation: www.circulationaha.org Ponikowski et al. Ventilation in Chronic Heart Failure, February 20, 2001, the teachings of which are incorporated by reference. Ventilatory efficiency, VO₂ and O₂ pulse were assessed up to the anaerobic threshold at baseline and again at eight weeks. The results for the first group of0 test patients (2 females and 13 males) are summarized in Table I. "R" designates D-ribose. Each patient acted as his or her control, that is, results after ribose administration were compared to baseline results. VO₂ efficiency is the O₂ uptake per unit time. O₂ pulse is a measurement of the heart stroke volume.

**TABLE I**

| Ventilatory efficiency | | VO₂ efficiency | | O₂ pulse | |
|---|---|---|---|---|---|
| Pre-R | Post-R | Pre-R | Post-R | Pre-R | Post-R |
| 50.6+/-9.8 | 41.6+/-6.4 | 1.00+/-0.28 | 1.30+/-0.28 | 7.45+/-1.8 | 9.04+/-1.9 |
| (P<0.01) | | (P<0.028) | | (P<0.05) | |

Results show that the administration of D-ribose improved VE by about 20% in this study. Note that the improvement in VO₂ was higher, possibly confirming the earlier observation that a "point in time" measurement may not be an accurate assessment of pulmonary function. It was also found that several of the patients were reclassified into a higher, that is, less severe, Class.

### B. Detailed results of representative patients.

A 59 year old male, normal weight, was diagnosed with blockage of the coronary arteries with stable angina, not yet progressing to congestive heart failure. A CAT scan showed no myocardial infarction. Using a treadmill, with incremental increase in grade, his V_{O2} max and V_{CO2} were determined. Following eight weeks of ribose administration of five grams four times a day, he was retested under the same conditions. Plotting a regression analysis of V_{O2} versus log V, the VE slope decreased from 60.2 to 45.5. It is considered that a slope of 36.9 or below indicates impairment of ventilatory efficiency. Therefore, while this patient was not in the normal range of ventilatory efficiency, improvement was marked.

A second patient, a 77 year old male of normal weight, self administered five grams of ribose four times a day for eight weeks. At the beginning of the study, his VE slope was 55.7 following nine minutes of treadmill simulation exercise. At the end of the study, his VE slope had decreased to 45.2. This patient also was tested on the step test. The initial test was rated as "good" and the second test was subjectively considered to be "great."

A third patient, a 72 year old obese woman, was on nasal oxygen and was tested with drug-driven simulated exercise. After administration of five grams of ribose four times daily for eight weeks, her VE slope decreased from 63.0 to 35.2 and the time of simulated exercise was increased from 7.43 minutes to 11.44. minutes.. She was able to discontinue the oxygen. Although her VE was now in the normal range, the test results, although improved were not subjectively rated as "good".

### 2. Ventilatory efficiency in rheumatoid lung.

Autoimmune diseases such as rheumatoid arthritis and sarcoidosis eventually result in poor pulmonary function. Exposure to toxins may cause similar deficits in breathing ability. These conditions are chronic and patients are advised to exercise as much as possible, but many are not willing to do so because of fatigue, shortness of breath and wheezing.

A 53-year old woman developed rheumatoid arthritis in the 1970's. By 1988, she began to show symptoms of rheumatoid lung, began the use of rescue inhalers such as Albuterol® and was hospitalized for respiratory distress three times in the next five years. At that point, she was prescribed a steroid inhaler, Advair®, which relieved her symptoms considerably, although she still required a rescue inhaler several times per week. In 2002, she began the administration of ribose, approximately five grams two to three times a day. Within a month, she was able to discontinue the use of the rescue inhaler and to exercise more.

## Claims

1. D-Ribose for use in improving ventilatory efficiency in subjects suffering from autoimmune disease resulting in poor pulmonary function.

2. The D-ribose for use according to claim 1, wherein the autoimmune disease is rheumatoid lung.

## Patentansprüche

1. D-Ribose für die Verwendung bei der Verbesserung der Atmungseffizienz bei Personen, die an einer Autoimmunerkrankung leiden, die zu einer schlechten Lungenfunktion führt.

2. D-Ribose für die Verwendung nach Anspruch 1, wobei die Autoimmunerkrankung eine rheumatische Lunge ist.

## Revendications

1. D-ribose pour une utilisation destinée à améliorer l'efficacité ventilatoire chez des sujets souffrant d'une maladie auto-immune aboutissant à une fonction pulmonaire médiocre.

2. D-ribose pour l'utilisation selon la revendication 1, dans lequel la maladie auto-immune est le poumon rhumatoïde.
